# EUROPEAN PATENT SPECIFICATION

(11) **EP 4 346 614 B1**
(45) Date of publication and mention of the grant of the patent: **17.06.2026**
(21) Application number: 22728646.5
(22) Date of filing: 25.05.2022
(51) Int. Cl.: A61B 8/06, A61B 8/08, G01S 15/89

(54) **SUPER RESOLUTION ULTRASOUND IMAGING**
ULTRAHOCHAUFLÖSENDE ULTRASCHALLBILDGEBUNG
IMAGERIE ULTRASONORE À SUPER RÉSOLUTION

(30) Priority: 02.06.2021 US 202117303574; 11.06.2021 EP 21179070
(43) Date of publication of application: 10.04.2024
(73) Proprietor: Danmarks Tekniske Universitet, 2800 Kongens Lyngby (DK)
(72) Inventor: JENSEN, Jørgen Arendt, 2800 Kongens Lyngby (DK); SCHOU, Mikkel, 2800 Kongens Lyngby (DK)
(74) Representative: Guardian IP Consulting I/S
(86) International application number: PCT/EP2022/064226
(87) International publication number: WO 2022/253673

(56) References cited:
- WO-A1-2020/083679
- US-A1- 2013 281 846
- YU JAESOK ET AL: "Super-resolution ultrasound imaging method for microvasculature in vivo with a high temporal accuracy", vol. 8, no. 1, 17 September 2018 (2018-09-17), XP055861054, Retrieved from the Internet <URL:http://www.nature.com/articles/s41598-018-32235-2> DOI: 10.1038/s41598-018-32235-2
- LEOW CHEE HAU ET AL: "3-D Microvascular Imaging Using High Frame Rate Ultrasound and ASAP Without Contrast Agents: Development and Initial In Vivo Evaluation on Nontumor and Tumor Models", IEEE TRANSACTIONS ON ULTRASONICS, FERROELECTRICS, AND FREQUENCY CONTROL, IEEE, USA, vol. 66, no. 5, 1 May 2019 (2019-05-01), pages 939 - 948, XP011721147, ISSN: 0885-3010, [retrieved on 20190424], DOI: 10.1109/TUFFC.2019.2906434
- SONG PENGFEI ET AL: "Ultrasound Small Vessel Imaging With Block-Wise Adaptive Local Clutter Filtering", IEEE TRANSACTIONS ON MEDICAL IMAGING, IEEE, USA, vol. 36, no. 1, 1 January 2017 (2017-01-01), pages 251 - 262, XP011638275, ISSN: 0278-0062, [retrieved on 20161230], DOI: 10.1109/TMI.2016.2605819
- ARTUL SUHEIL ET AL: "Superb Microvascular Imaging: Added Value and Novel Applications", vol. 7, 28 December 2017 (2017-12-28), pages 45, XP055861123, Retrieved from the Internet <URL:https://www.ncbi.nlm.nih.gov/pmc/articles/PMC5765119/pdf/JCIS-7-45.pdf> DOI: 10.4103/jcis.JCIS_79_17

## Description

### TECHNICAL FIELD

The following generally relates to ultrasound and more particularly to super resolution ultrasound imaging.

### BACKGROUND

The literature indicates that the diffraction limit of conventional ultrasound imaging is about half a wavelength. Clinical ultrasound imaging applications have used wavelengths between 200 microns (200 µm) and one millimeter (1 mm), which precludes imaging of smaller structures with diameters less than 100 µm such as the microvasculature. Super resolution ultrasound imaging produces an image with a resolution that is beyond the diffraction limit of conventional ultrasound imaging, providing for visualization of microvascular vessels with diameters down to 8 µm.

An example of an ultrasonic imaging technique is disclosed in US 2013/0281846, which describes an ultrasonic diagnostic apparatus that scans the inside of an object administered with a contrast agent with ultrasonic waves.

Another example is described in the scientific paper "Super-resolution ultrasound imaging method for microvasculature in vivo with a high temporal accuracy", SCIENTIFIC REPORTS, vol. 8, no. 1, by Yu Jaesok et al., This prior art document discloses an imaging sequence and signal processing approach for super-resolution ultrasound imaging to improve temporal resolution by employing deconvolution and spatio-temporal-interframe-correlation based data acquisition.

Unfortunately, super resolution ultrasound imaging is an invasive procedure that includes continuously administering a microbubble based contrast agent intravenously during the examination. In general, with super resolution ultrasound imaging centers of the individual microbubbles are tracked over time in ultrasound images. Since individual microbubbles are tracked, the density of the microbubbles in the administered contrast agent is sparse as it might not be possible to distinguish individual microbubbles in dense populations. However, with a sparse density it can take several minutes (e.g., 7 minutes) for the microbubbles to disperse through the full circulation.

During the examination, the subject needs to remain still with a precision of around 50 µm for several minutes. Unfortunately, it is difficult for a person to remain that still for several minutes. In addition to voluntary movement, the acquisition is also subject to effects from involuntary movement such as breathing, the heart beating, etc. Unlike a conventional scan that can be completed in a single breath hold, it is not reasonable to ask the subject to hold their breath for a several minute examination to mitigate effects from breathing. To compensate for both voluntary and involuntary movement, the processing includes motion compensation. The long acquisition time also precludes real time display.

The microbubbles in a microbubble based contrast agent tend to be fragile and burst if exposed to certain levels of acoustic pressure. To prevent them from bursting, acquisition sequences have been limited to a mechanical index (MI) (which is used as the index of cavitation bio-effects) of 0.05 to 0.20. The United States Food and Drug Administration (US FDA) limits diagnostic ultrasound imaging to an MI of 1.9. Unfortunately, the lower MI used with super resolution ultrasound imaging reduces the transmitted energy and thus the signal-to-noise ratio (SNR) and penetration depth.

The scientific article "3-D Microvascular Imaging Using High Fraine Rate Ultrasound and ASAP Without Contrast Agents: Development and Initial In Vivo Evaluation on Nontumor and Tumor Models" by Chee Hau Leow et al., IEEE Transactions on Ultrasonics, Ferroelectrics, and Frequency Control, Vol 66, No. 5, discloses an approach for micro-vascular ultrasound imaging. While this prior art technique can be implemented in real-time and may be suitable for microvascular imaging applications where super-resolved pixels beyond diffraction limit is not required.

In view of at least the foregoing, there is an unresolved need for an improved approach to super resolution ultrasound imaging.

### SUMMARY

Aspects of the application address the above matters, and others. The invention is defined in the independent claims.

In one aspect, an apparatus includes a processing pipeline. The processing pipeline includes a stationary structure motion corrector configured to motion correct stationary structure in a series of ultrasound images for subject motion, wherein the series of ultrasound images includes the stationary structure and flowing structure. The processing pipeline further includes a stationary structure remover configured to remove the stationary structure from the motion corrected series of ultrasound images thereby producing flow images of the flowing structure. The processing pipeline further includes a flowing structure detector configured to detect peaks of flow of the flowing structure in the flow images over time to generate images of detected flow peaks. The images of detected flow peaks are accumulated over time to generate a super resolution ultrasound image.

The term "super resolution ultrasound imaging" as used herein refers to a process for creating ultrasound images that have a resolution beyond the diffraction limit of the ultrasound waves used for creating the ultrasound images, i.e. a resolution high enough to distinguish structures smaller than half the wavelength of the ultrasound waves used for creating the ultrasound images. The term "super resolution ultrasound image" as used herein refers to an ultrasound image produced by super resolution ultrasound imaging, i.e. to an ultrasound image having a resolution high enough to distinguish structures smaller than half the wavelength of the ultrasound waves used for creating the ultrasound image, such as structures smaller having a size smaller than 100 µm, such as smaller than 50 µm, such as smaller than 10 µm.

The apparatus may include one or more processors programmed or otherwise configured to implement the processing pipeline. The one or more processors may include a central processing unit (CPU), a graphics processing unit (GPU), a microprocessor, and/or the like.

In some embodiments, the apparatus includes an imaging system configured for ultrasound imaging, including super resolution ultrasound imaging, e.g., of at least the microvasculature of a subject. In some embodiments, the imaging system includes a probe and a console operatively coupled to the probe. The probe may include a transducer array with one or more transducer elements, e.g. a one dimensional (1-D), matrix or row-column array, a linear, curved or otherwise shaped, a fully populated or sparse, etc. array. The transducer elements may be configured to convert excitation electrical pulses into an ultrasound pressure field and to convert received ultrasound pressure fields (echoes) into an electrical (e.g., a radio frequency (RF)) signal. The echoes are generated in response to the transmitted pressure field interacting with matter, e.g., erythrocytes, tissue, etc.

In some embodiments, the console includes transmit circuitry configured to generate the excitation electrical pulses that excite the transducer elements and receive circuitry configured to receive and, optionally, condition and/or preprocess the electrical, e.g. RF, signals produced by the transducer elements.

In some embodiments, the apparatus includes circuitry, e.g. a processing unit, configured to implement to process the received electrical signals, in particular the received RF signals, to create a series of ultrasound images. In some embodiments, the apparatus further includes circuitry, e.g. the same or a separate processing unit, configured to implement a processing pipeline for processing the series of ultrasound images to generate the high resolution ultrasound image, in particular the super resolution ultrasound image, as described herein. It will be appreciated that the creation of the series of ultrasound images from the received electrical signals and the generation of high resolution ultrasound images, in particular of super resolution ultrasound images, from the series of ultrasound images may be implemented as part of a single processing pipeline which may be implemented by a single processor. Alternatively, the different acts may be distributed between multiple processors. In some embodiments, the processor(s) implementing the processing pipeline, or a part of the processing pipeline, are included in the console. In some embodiments, the processing pipeline, or a part of the processing pipeline is part of a computing apparatus separate from the console. With this embodiment, RF signals stored in memory and/or beamformed images stored in memory of the computing apparatus or received from the console (e.g. via a suitable wired or wireless connection) can be loaded and processed with the processing pipeline to generate the images of peak detections as described herein.

In some embodiments, the processing pipeline is configured to generate a series of high resolution images from the received electrical, e.g. RF, signals, to motion correct the series of high resolution images for stationary structure motion due to subject movement (voluntary and/or involuntary), to remove the stationary structure from the motion corrected series of high resolution images to produce flow images of the flowing structure (e.g., erythrocytes), to detect positions of flow peaks of the flowing structure in the flow images, and to accumulate the images of peak detections over time. The apparatus may further include a display and be configured to display the generated high resolution ultrasound images. The display may be included in the console or in a separate computing apparatus. In particular, the series of high resolution images may be a series of super resolution ultrasound images.

In some embodiments, the processing pipeline is configures to produce an image of a microvasculature with diameters less than 100 µm, e.g., down to 2 µm. Accordingly, the generated high resolution images will also be referred to as super resolution images, as they may have a resolution high enough to distinguish structures having dimensions smaller than half the wavelength of the ultrasound waves emitted by the transducer array. The density of erythrocytes in circulation is ≈ 5 million per mm³ and the approach does not track individual erythrocytes, but rather their flow as a whole with examination times in the seconds, allowing for real time display, relaxing motion compensation requirements and subject movement constraints, and allowing for an MI up to the US FDA limit for diagnostic ultrasound imaging and thus not compromising SNR or depth penetration. In addition, the approach described herein is non-invasive, unlike microbubble based approaches.

Accordingly, the flowing structure consists of flowing biological material of the subject body under investigation without any added contrast agent or added microbubbles. In particular, the flowing biological material may include one or more components of the subject's blood, in particular erythrocytes. The stationary structure may include stationary biological material, such as tissue, of the subject body. The flowing structure is structure flowing relative to the stationary structure, i.e. the stationary structure is stationary relative to the flowing structure. It will be appreciated, however, that the stationary structure may be moving during the data acquisition, e.g. due to voluntary or involuntary movement of the subject's body.

In some embodiments, the apparatus includes a flow tracker configured to link the detect peaks of flow across the images of detected flow peaks, creating tracks of flow. In some embodiments, the apparatus includes a velocity estimator configured to estimate velocity information based on the tracks of flow. In some embodiments, the apparatus includes a vessel width estimator configured to estimate a width of a vessel based on the super resolution ultrasound image. The flow tracker, the velocity estimator and/or the vessel width estimator may be implemented by the processor implementing the processing pipeline, e.g. as part of the processing pipeline or separate therefrom. Alternatively, the flow tracker, the velocity estimator and/or the vessel width estimator may be implemented by a separate processor.

In another aspect, a computer-implemented method as defined in claim 11 is disclosed. The method includes motion correcting stationary structure in a series of ultrasound images for subject motion, wherein the series of ultrasound images includes the stationary structure and flowing structure. The method further includes removing the stationary structure from the motion corrected series of ultrasound images thereby producing flow images of the flowing structure. The method further includes detecting peaks of flow of the flowing structure in the flow images over time to generate images of detected flow peaks. The method further includes accumulating the images of detected flow peaks over time to generate a high resolution ultrasound image, in particular a super resolution ultrasound image.

In yet another aspect, a computer program includes instructions that when executed by a computer cause the computer to perform the steps of the method disclosed above and in the following. The computer program may be implemented as a computer-readable storage medium storing the instructions or as a data signal encoding the instructions.

According to another aspect, disclosed herein are embodiments of a data processing system configured to perform the acts of the method described herein. In particular, the data processing system may have stored thereon program code adapted to cause, when executed by the data processing system, the data processing system to perform the steps of the method described herein. The data processing system may be embodied as a single computer or as a distributed system including multiple computers, e.g. a client-server system, a cloud based system, etc.

Those skilled in the art will recognize still other aspects of the present application upon reading and understanding the attached description.

### BRIEF DESCRIPTION OF THE DRAWINGS

The application is illustrated by way of example and not limited by the figures of the accompanying drawings, in which like references indicate similar elements and in which:
FIGURE 1 diagrammatically illustrates an example imaging system, in accordance with an embodiment(s) herein;
FIGURE 2 diagrammatically illustrates an example processing pipeline for the system of FIGURE 1, in accordance with an embodiment(s) described herein;
FIGURE 3 diagrammatically illustrates a variation of the processing pipeline of FIGURE 2 that includes a flow tracker, in accordance with an embodiment(s) described herein;
FIGURE 4 diagrammatically illustrates another variation of the processing pipeline of FIGURE 1 that includes a flow tracker and a velocity estimator, in accordance with an embodiment(s) described herein;
FIGURE 5 diagrammatically illustrates a variation of the system of FIGURE 1 that includes a vessel width estimator, in accordance with an embodiment(s) described herein;
FIGURE 6 depicts a portion of super resolution ultrasound image with indicator superimposed thereover identifying a location for a vessel width estimation;
FIGURE 7 depicts a graph of vessel widths estimated by the vessel width estimator of FIGURE 5 for the vessels indicated in FIGURE 6;
FIGURE 8 illustrates an example method, in accordance with an embodiment(s) herein;
FIGURE 9 depicts accumulation of the super resolution ultrasound image generated by the system of FIGURE 1 after t₁ seconds;
FIGURE 10 depicts the accumulation of the super resolution ultrasound image generated by the system of FIGURE 1 after tᵢ seconds;
FIGURE 11 depicts the accumulation of the super resolution ultrasound image generated by the system of FIGURE 1 after tₙ seconds;
FIGURE 12 depicts a super resolution ultrasound image generated by the system of FIGURE 1;
FIGURE 13 depicts a computed tomography image of the same anatomy that is in the image of FIGURE 12; and
FIGURE 14 depicts a fusion of the super resolution ultrasound image of FIGURE 12 and the computed tomography image of FIGURE 13.

### DETAILED DESCRIPTION

The following describes a non-invasive super resolution ultrasound imaging approach that mitigates one or more of the above-noted shortcoming of microbubble based super resolution ultrasound imaging. In general, this approach includes a processing pipeline configured to process ultrasound images to create images of only/mainly flowing structure such as erythrocytes and display an accumulation of peaks of flowing structure detected over time in the images to produce a super resolution ultrasound image.

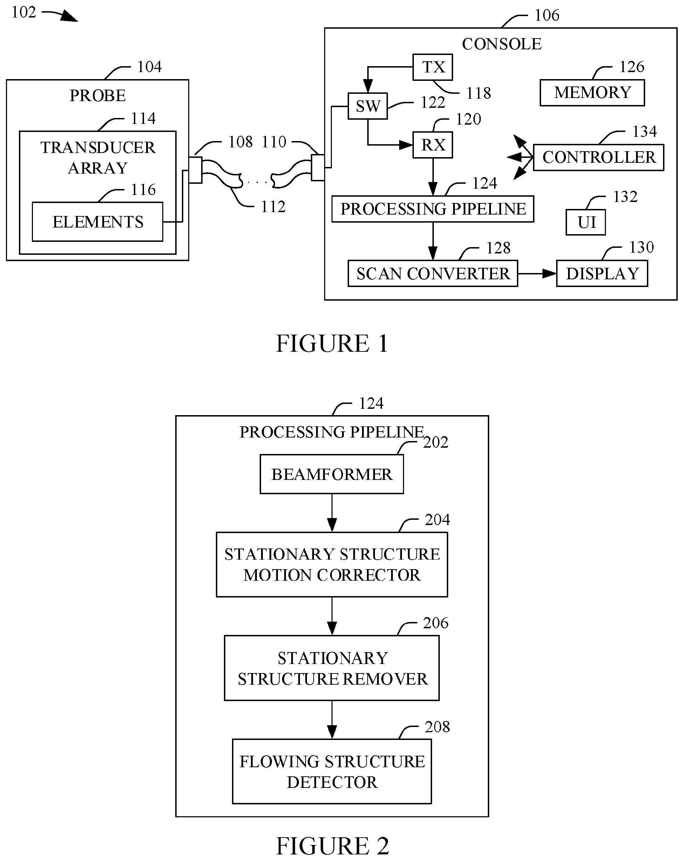

FIGURE 1 illustrates an example imaging system 102 configured for ultrasound imaging, including super resolution ultrasound imaging, e.g., of at least the microvasculature of a subject. The imaging system 102 includes a probe 104 and a console 106, which interface with each other through suitable complementary hardware (e.g., electromechanical connectors 108 and 110 and a cable 112 as shown, etc.) and/or a wireless interface (not visible).

The probe 104 includes a transducer array 114 with one or more transducer elements 116. The transducer array 114 includes a one dimensional (1-D), matrix or row-column array, a linear, curved or otherwise shaped, a fully populated or sparse, etc. array. The transducer elements 116 are configured to convert excitation electrical pulses into an ultrasound pressure field and to convert received ultrasound pressure fields (echoes) into an electrical (e.g., a radio frequency (RF)) signal. The echoes are generated in response to the transmitted pressure field interacting with matter, e.g., erythrocytes, tissue, etc.

The console 106 includes transmit circuitry (TX) 118 configured to generate the excitation electrical pulses that excite the transducer elements 116 and receive circuitry (RX) 120 configured to receive the RF signals produced by the transducer elements 116. In one embodiment, the RX 120 (or other circuitry) is configured to also condition or preprocess the RF signal, e.g., amplify, digitize, etc. In the illustrated embodiment, a switch (SW) 122 is configured to switch between the TX 118 and RX 120 for transmit and receive operations. In an alternative embodiment, separated switches are employed.

In one embodiment, the TX 118 and RX 120 are controlled to concurrently acquire all image lines in each emission. For example, a subset (i.e. one or a subgroup) of the elements can be excited to simultaneously produce pressure fields that together emit a focused beam, and all of the elements can be used to receive echoes. This can be repeated for multiple different subsets, where each emission/reception provides data to generate a lower resolution image, and a higher resolution image can be generated by combining lower resolution images. An example of a suitable sequence is described in Jensen et al., "Synthetic aperture ultrasound imaging," Ultrasonics, vol. 44, pp. e5-e15, 2006. Another example using plane waves is mentioned in Tanter et al., "Ultrafast imaging in biomedical ultrasound, IEEE Transactions on Ultrasonics, Ferroelectrics, and Frequency Control, 2014, 61, 1, pp. 102-119.

In another embodiment, the TX 118 and RX 120 are controlled to sequentially acquire one image line at a time. For instance, a subset (i.e. one or a subgroup) of the elements can be excited to simultaneously produce pressure fields that together to emit a focused beam, and the subset can then receive a single line of echoes in response thereto. This is repeated for multiple different subsets to sequentially acquire multiple image lines which together form an image. It is to be understood that the above three sequences are non-limiting and other acquisition sequences, including known sequences for 2-D, 3-D and/or 4-D imaging, are also contemplated herein.

The console 106 further includes a processing pipeline 124. The processing pipeline 124 can include one or more processors (e.g., a central processing unit (CPU), graphics processing unit (GPU), a microprocessor, etc.) configured to execute computer readable instructions encoded or embedded on computer readable storage medium such as memory 126 to perform the acts describe herein. In general, the processing pipeline 124 is configured to process the RF signals to create a set of images and to combine the images to generate a super resolution ultrasound image.

As described in greater detail below, in one instance the processing pipeline 124 generates a series of super resolution images with the RF signals, motion corrects the series of super resolution images for stationary structure motion due to subject movement (voluntary and/or involuntary), removes the stationary structure from the motion corrected series of super resolution images to produce flow images of the flowing structure (e.g., erythrocytes), detects positions of flow peaks of the flowing structure in the flow images, and accumulates the images of peak detections over time.

It one instance, this may include producing an image of the microvasculature with diameters less than 100 µm, e.g., down to 2 µm. The density of erythrocytes in circulation is ≈ 5 million per mm³ and the approach does not track individual erythrocytes, but rather their flow as a whole with examination times in the seconds, allowing for real time display, relaxing motion compensation requirements and subject movement constraints, and allowing for an MI up to the US FDA limit for diagnostic ultrasound imaging and thus not compromising SNR or depth penetration. In addition, the approach described herein is non-invasive, unlike microbubble based approaches.

The console 106 further includes a scan converter 128 and a display 130. The scan converter 128 is configured to scan convert each image for display, e.g., by converting the images to the coordinate system of the display 130. A (2-D and/or 3-D) super resolution ultrasound image is then built up over time by adding the current processed image to the currently displayed image. The image can be further processed, e.g., smoothed with a Gaussian or other kernel to account for inaccuracies in the motion estimation, sub-sample registration, and/or sparseness of detection, and/or otherwise processed.

The console 106 further includes a user interface 132, which includes one or more input devices (e.g., a button, a touch pad, a touch screen, etc.) and one or more output devices (e.g., a display screen, a speaker, etc.). The console 106 further includes a controller 134 configured to control one or more of the transmit circuitry 118, the receive circuitry 120, the switch 122, the processing pipeline 124, the scan converter 128, the display 130, and/or the user interface 132.

FIGURE 2 diagrammatically illustrates a non-limiting example of the processing pipeline 124. The illustrated processing pipeline 124 receives, as input, the RF signals from the receive circuitry 120, and outputs images that when combined together provide a super resolution ultrasound image. In this example, the acquisition includes both stationary structure (e.g., organs, vessels, etc.) and flowing structure (e.g., erythrocytes), and the output images are images of detected peaks of the flowing structure.

The illustrated processing pipeline 124 includes a beamformer 202. The beamformer 202 is configured to beamform the RF signals and output a series of images. A non-limiting example of suitable beamforming is described in Stuart et al., "Real-time volumetric synthetic aperture software beamforming of row-column probe data," IEEE Trans. Ultrason., Ferroelec., Freq. Contr., April 8, 2021. Other beamforming approaches are contemplated herein.

The illustrated processing pipeline 124 further includes a stationary structure motion corrector 204. The stationary structure motion corrector 204 is configured to correct the images in the series for motion of stationary structure due to, e.g., subject involuntary and/or voluntary motion. In one instance, this includes rotating, translating, etc. images in the series to align the stationary structure image to image. A suitable approach, which estimates stationary structure motion from the envelope data using speckle correlation, is described in Trahey et al., "Angle independent ultrasonic detection of blood flow," IEEE Trans. Biomed. Eng., vol. BME-34, no. 12, pp. 965-967, 1987.

With this approach, for local motion estimation, each image is divided into a plurality of partially overlapping sub-regions, with one of the images being identified as a reference image. Examples of suitable sub-region sizes include, but are not limited to, 1x1 millimeters square (mm²), 10x10 mm², larger, smaller, non-square, etc. Examples of a suitable reference image the first B-mode image, the middle B-mode image, the last B-mode image, or other B-mode image of the series of B-mode images. In another instance, more than one reference image is utilized.

Another suitable approach, which determines both the axial and the lateral components using transverse oscillation, is described in US patent 6,859,659 B1 to Jensen, filed November 9, 2001, and entitled "Estimation of vector velocity". Another approach, which uses directional beamforming, is described in US patent 6,725,076 B1 to Jensen, filed January 25, 2002, and entitled "Vector velocity estimation using directional beam forming and cross-correlation".

The motion estimation can alternatively be performed for 3-D data as described in US patent application publication number 2016/0206285 to Christiansen et al., filed January 19, 2015, and entitled "3-d flow estimation using row-column addressed transducer arrays". With these approaches, the motion is determined relative to a reference image across the full acquisition. All images are then co-registered to the reference frame and aligned, e.g., via interpolation, such as spline interpolation, etc., for having a sequence of images aligned to the same spatial position over time.

Each sub-region in the reference image is cross-correlated with the corresponding sub-region in the other images, and motion in the axial and lateral directions is estimated. The estimated motion for each sub-region is assigned to a center (and/or other location) of the corresponding sub-region, and the collection of motion estimates for an image provides a discrete motion field through that image. The estimated displacements vary spatially and temporally. Motion can be estimated at any point in any image (i.e. space and/or time) using interpolation such a spline, etc. on the motion field. All the images are then compensated with this motion to align their content with the reference frame prior to the stationary structure remover 206.

The illustrated processing pipeline 124 further includes a stationary structure remover 206. The stationary structure remover 206 is configured to remove the stationary structure from the motion corrected images, producing images of just or mainly the flowing structure, e.g., the erythrocytes. In general, this can be achieved by subtracting the stationary structure from the images, which leaves the flowing structure.

Suitable approaches for removing the stationary structure include singular valued decomposition (SVD), filtering, Principal component analysis (PCA), and/or other approach. An example of a suitable approach is described in Demene et al., "Spatiotemporal clutter filtering of ultrafast ultrasound data highly increases Doppler and fUltrasound sensitivity," IEEE Trans. Med. Imag., vol. 34, no. 11, pp. 2271-2285, 2015.

With one approach, a Casarotti matrix can be formed from the data with a size of *N_{z}Nₓ x Nₜ,* where *N_{z}* is a number of samples in the axial direction, *Nₓ* is a number of lateral lines, and *Nₜ* is a number of time samples. The number of calculations to process is proportional to *O*((*N_{z}Nₓ*)*Nₜ*²)*.* The images can be divided into overlapping patches of pixels (e.g., 180 x 180 pixels) where a border of pixels overlaps adjacent patches. The singular values of these patches are then calculated. The singular values representing stationary structure can be set to zero. Singular values representing mostly noise can also be removed.

Images of flowing structure can then be reconstructed from the remaining non-zero singular values. An example of a suitable reconstructor is described in Baranger et al., "Adaptive spatiotemporal SVD clutter filtering for ultrafast Doppler imaging using similarity of spatial singular vectors," IEEE Trans. Med. Imag., vol. 37, no. 7, pp. 1574-1586, July 2018. In another instance, the stationary tissue structures are removed using other echo canceling approaches using a filter as described in Torp, "Clutter rejection filters in color flow imaging: A theoretical approach," IEEE Trans. Ultrason., Ferroelec., Freq. Contr., vol. 44, pp. 417-424, 1997, and/or other similar methods as described in Yu et al., "Eigen-based clutter filter design for ultrasound color flow imaging: A review," IEEE Transactions on Ultrasonics, Ferroelectrics, and Frequency Control, 57(5), 5456258, 2010.

The illustrated processing pipeline 124 further includes a flowing structure detector 208. The flowing structure detector 208 is configured to detect flowing structure (e.g. the erythrocytes) in the flow images. In one instance, the flowing structure detector 210 detects positions of local peaks in each of the patches. There will be many erythrocytes, and the complex summation of all scatterers within the vessel will have a peak position within the vessel. Individual erythrocytes cannot and need not be detected, unlike the microbubble approach, which requires detecting individual microbubbles.

For this approach, the envelope data can be log compressed and normalized with respect to the standard deviation of all the patches in the first image. Peaks above a threshold of minus 30 decibel (-30 dB) from the maximum in the first (or other) image are then identified as the location of the flow of interest (i.e. the erythrocytes). A subsample location of the peak can be found from interpolation, such as a polynomial interpolation around the peak position. Detections outside of a predetermined window (e.g., 100 x 100 pixels) about a central region of the patch can be discarded with only detections inside the window being kept. This can be performed for all patches and for all frames in the sequence.

Again, the output of the processing pipeline 124 is a series of images of detected peaks of flow, which, in this example, corresponds to the flowing erythrocytes. As discussed herein, the scan converter 128 scan converts each image for display, and a super resolution ultrasound image is generated on the display by accumulating the individual images over time.

Variations are discussed next.

In one variation, the processing pipeline 124 is part of a different computing apparatus. With this embodiment, RF signals stored in memory and/or beamformed images stored in memory can be loaded and processed with the processing pipeline 124 to generate the images of peak detections as described herein. Such an apparatus can include a scan converter and display for constructing and visually displaying a super resolution ultrasound image. Alternatively, or additionally, the images of peak detections can be conveyed to the system 102, another ultrasound system, and/or another computer apparatus to construct and visually display a super resolution ultrasound image.

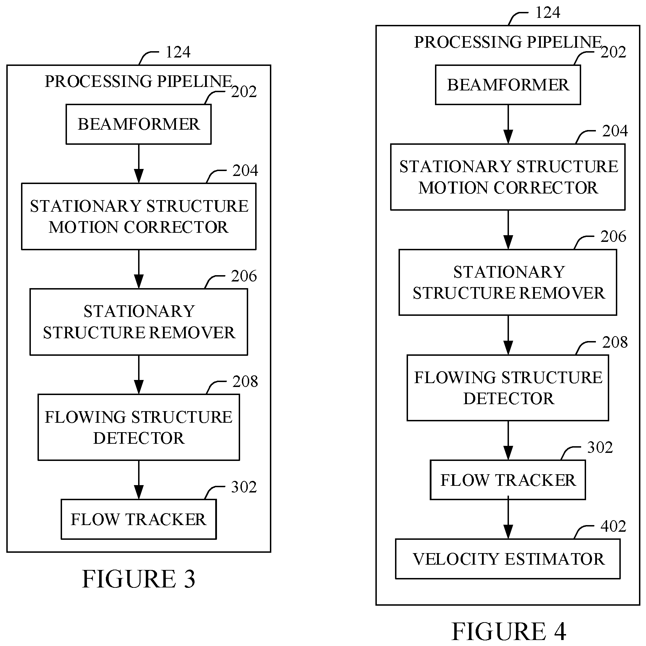

In another variation, the processing pipeline 124 further includes a flow tracker 302, e.g. as shown in FIGURE 3. The flow tracker 302 is configured to generate tracks that link flowing structure from frame to frame. Suitable approaches include, but are not limited to, nearest-neighbor, multi-frame data structure, dynamic programming, combinatorial, multi hypothesis, explicit motion models (e.g. Kalman filtering), learning-based, and/or other techniques. Color coding and/or other image processing can be used to visually show flow information such as flow direction, volume flow, and/or derived quantities like pressure gradients, resistive index, turbulence and/or perfusion, etc. An example of tracking flow is described in US application serial number 16/929,398 to Jensen et al., filed on July 15, 2020, and entitled "Ultrasound Super Resolution Imaging".

In another variation, the processing pipeline 124 further includes the flow tracker 302 and a velocity estimator 402, e.g. as shown in FIGURE 4. The velocity estimator 402 is configured to process track locations to estimate velocity such as mean velocity, peak velocity, etc. By way of non-limiting example, the velocity estimator 402 can be configured to determine a time derivative of track locations, which yields both the axial and lateral velocities. Color coding and/or other image processing can be used to visually show flow velocity, in addition or in alternative, to flow direction and/or other flow information. An example of estimating velocity from flow tracks is described in US application serial number 16/929,398 to Jensen et al., filed on July 15, 2020, and entitled "Ultrasound Super Resolution Imaging."

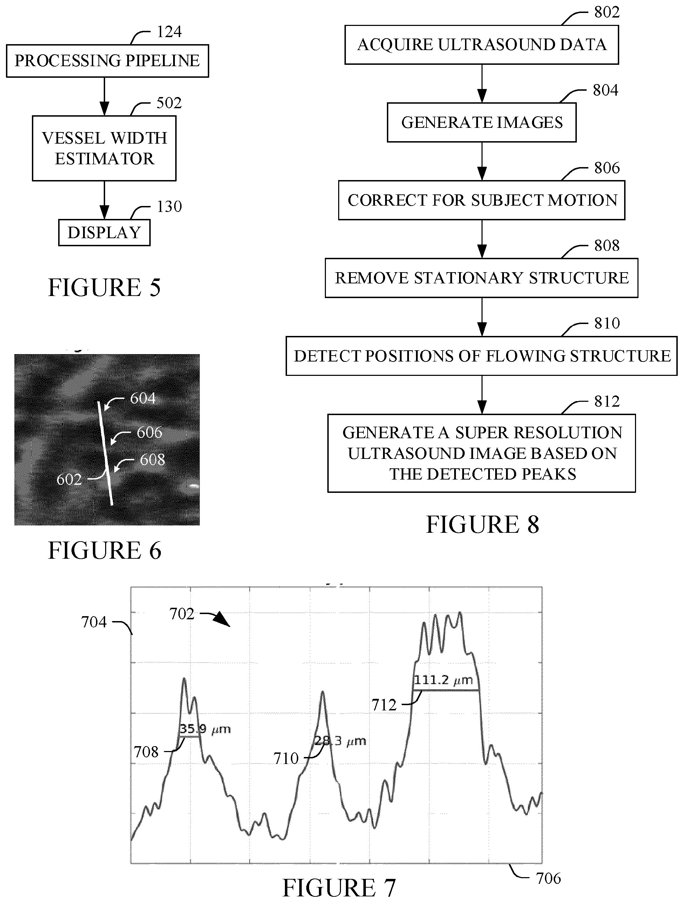

In another variation, the system 102 includes a vessel width estimator 502, e.g. as shown in FIGURE 5. The vessel width estimator 502 is configured to estimate a width of a vessel based on the super resolution ultrasound image. In one instance, a user, via the UI 132 and/or otherwise, indicates on the image where they would like to estimate vessel widths, and the widths are estimated and displayed. An example is shown in FIGURES 6 and 7, which are described next.

FIGURE 6 shows a portion of a super resolution ultrasound image with a user placed indicator 602 across several vessels of interest, including a vessel 604, a vessel 606 and a vessel 608. The illustrated indicator 602 is a straight line. However, other shapes are contemplated herein. FIGURE 7 shows a graph 702 of density of detections (a first axis 704) as a function of spatial distance on the indicator 602 (a second axis 706). In this example, the estimated widths 708, 710 and 712 represent -3 dB widths respectively of vessels 604, 606 and 608.

In another variation, another component(s) of blood (e.g., leukocytes, thrombocytes, plasma, etc.) is tracked using the approach described herein.

FIGURE 8 illustrates an example method in accordance with an embodiment herein.

The ordering of the following acts is for explanatory purposes and is not limiting. As such, one or more of the acts can be performed in a different order, including, but not limited to, concurrently. Furthermore, one or more of the acts may be omitted and/or one or more other acts may be added.

At 802, ultrasound data is acquired, as described herein and/or otherwise.

At 804, the ultrasound data is beamformed to produce images, as described herein and/or otherwise.

It is to be appreciated that acts 802 and 804 can be omitted. For example, in another instance previously acquired and stored images are retrieved from memory.

At 806, the stationary structure in the images is corrected for subject motion, as described herein and/or otherwise.

At 808, the stationary structure is removed from the images producing flow images, as described herein and/or otherwise.

At 810, positions of peaks of the flowing structure are detected in the flow images, as described herein and/or otherwise.

At 812, a super resolution ultrasound image is generated and displayed by summing images of the detected peaks of flow, as described herein and/or otherwise.

Optionally, flow information such as direction, volume flow, etc., and/or derived quantities such as pressure gradients, resistive index, turbulence and/or perfusion, etc. can be estimated and visualized, as described herein and/or otherwise.

Optionally, velocity information such as mean velocity, a peak velocity, etc. can be estimated and visualized, as described herein and/or otherwise.

In one embodiment, the above is implemented for 2-D ultrasound imaging. Alternatively, or additionally, the above is implemented for 3-D ultrasound imaging, e.g., to yield all the quantities in a volume using e.g., matrix probes and/or row-column arrays for volumetric imaging.

The above may be implemented by way of computer readable instructions, encoded or embedded on the memory 126 (i.e., the computer readable storage medium, which excludes transitory medium), which, when executed by a computer processor(s) cause the processor(s) to carry out acts described herein. Additionally, or alternatively, at least one of the computer readable instructions is carried by a signal, carrier wave or other transitory medium (which is not computer readable storage medium).

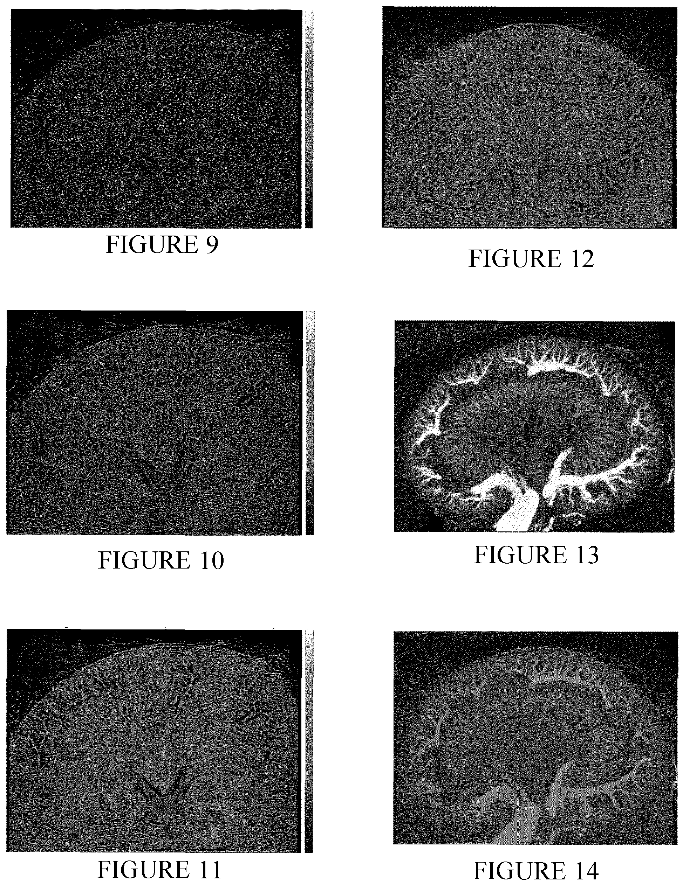

FIGURES 9-12 show a time evolution of a super resolution ultrasound image at t₁ seconds, tᵢ seconds, and tₙ seconds. In this example, larger vessels are depicted in FIGURE 9, but smaller vessels with diameters from 100 to 200 µm are barely visible. This improves progressively over time as information from more images is added as shown through FIGURES 10 and 11. In FIGURE 10, vessels with a diameter of 100 µm are discernible. In FIGURE 12, vessels with a diameter less than 100 µm such as 28 µm and paired arteries and veins are also distinguishable.

FIGURES 12-14 show a comparison of the approach described herein with respect to an image generated from a contrast agent based micro-computed tomography (CT) scan. Both the super resolution ultrasound image and the CT image are images of a kidney. The FIGURE 12 shows a super resolution ultrasound image. This image clearly depicts the long straight vasa recta of the medulla with a diameter of ≈20 µm and paired arcuate arteries and veins on the border between the cortex and medulla.

FIGURE 13 shows a CT image of the same anatomy after post-processing with a maximum intensity projection (MIP) algorithm in which only the voxels with the highest attenuation value are projected to produce an image. FIGURE 14 shows the fusion of the super resolution ultrasound image of FIGURE 12 and the CT image of FIGURE 13. FIGURE 14 shows a good correspondence between structures in the super resolution ultrasound image and the micro CT image.

The application has been described with reference to various embodiments. Modifications and alterations will occur to others upon reading the application. It is intended that the invention be construed as including all such modifications and alterations, including insofar as they come within the scope of the appended claims.

## Claims

1. An apparatus (106), comprising:
a processing pipeline (124), including:
a stationary structure motion corrector (204) configured to motion correct stationary structure in a series of ultrasound images for subject motion, wherein the series of ultrasound images includes the stationary structure and flowing structure;
a stationary structure remover (206) configured to remove the stationary structure from the motion corrected series of ultrasound images thereby producing flow images of the flowing structure; and
a flowing structure detector (208) configured to detect peaks of flow of the flowing structure in the flow images over time to generate images of detected flow peaks,
wherein the images of detected flow peaks are accumulated over time to generate a super resolution ultrasound image,
**characterized in that** the flowing structure consists of flowing biological material of a subject body under investigation without any added contrast agent or added microbubbles.

2. The apparatus of claim 1, wherein the flowing structure includes erythrocytes, the detected peaks correspond to flow of the erythrocytes, and the super resolution ultrasound image visually shows the vasculature.

3. The apparatus of any of claims 1 to 2, wherein the stationary structure motion corrector aligns the stationary structure across the series of ultrasound images to compensate for the subject motion.

4. The apparatus of claim 3, wherein the stationary structure motion corrector employs motion fields to align the stationary structure temporally, spatially or temporally and spatially.

5. The apparatus of any of claims 3 to 4, wherein the stationary structure remover subtracts the stationary structure from the motion compensated series of ultrasound images to produce the flow images.

6. The apparatus of any of claims 1 to 5, wherein the processing pipeline is configured to generate the super resolution ultrasound image in 1 to 10 seconds.

7. The apparatus of any of claims 1 to 6, further comprising:
a flow tracker (302) configured to link the detect peaks of flow across the images of detected flow peaks, creating tracks of flow.

8. The apparatus of claim 7, wherein the flow tracker is configured to determine flow information based on the tracks of flow.

9. The apparatus of any of claims 7 to 8, further comprising:
a velocity estimator (402) configured to estimate velocity information based on the tracks of flow.

10. The apparatus according to any one of the preceding claims, comprising a transducer array with one or more transducer elements for emitting ultrasound waves used to create the series of ultrasound images, and wherein the super resolution ultrasound image has a resolution high enough to distinguish structures having dimensions smaller than half the wavelength of the ultrasound waves emitted by the transducer array.

11. A computer-implemented method, comprising:
motion correcting stationary structure in a series of ultrasound images for subject motion, wherein the series of ultrasound images includes the stationary structure and flowing structure;
removing the stationary structure from the motion corrected series of ultrasound images thereby producing flow images of the flowing structure;
detecting peaks of flow of the flowing structure in the flow images over time to generate images of detected flow peaks; and
accumulating the images of detected flow peaks over time to generate a super resolution ultrasound image,
**characterized in that** the flowing structure consists of flowing biological material of a subject body under investigation without any added contrast agent or added microbubbles.

12. The method of claim 11, wherein the flowing structure includes erythrocytes, the detected peaks correspond to the erythrocytes, and the super resolution ultrasound image visually shows microvasculature.

13. The method of any of claims 11 to 12, wherein the motion correcting includes aligning the stationary structure across the series of ultrasound images to compensate for the subject motion.

14. The method of any of claims 11 to 13, wherein the super resolution ultrasound image has a resolution high enough to distinguish structures having dimensions smaller than half the wavelength of the ultrasound waves used for generating said series of ultrasound images.

15. A computer-readable storage medium having stored instructions that when executed by a computer cause the computer to perform the steps of the method according to any one of claims 11 through 14.

## Patentansprüche

1. Einrichtung (106), umfassend:
eine Verarbeitungspipeline (124), beinhaltend:
einen Bewegungskorrektor (204) für stationäre Strukturen, der dazu konfiguriert ist, eine Bewegung einer stationären Struktur in einer Reihe von Ultraschallbildern hinsichtlich einer Subjektbewegung zu korrigieren, wobei die Reihe von Ultraschallbildern die stationäre Struktur und eine strömende Struktur beinhaltet;
einen Entferner (206) stationärer Strukturen, der dazu konfiguriert ist, die stationäre Struktur aus der bewegungskorrigierten Reihe von Ultraschallbildern zu entfernen, wodurch Strömungsbilder der strömenden Struktur erzeugt werden; und
ein Strömungsstrukturdetektor (208), der dazu konfiguriert ist, Strömungsspitzen der strömenden Struktur in den Strömungsbildern über die Zeit zu erfassen, um Bilder erfasster Strömungsspitzen zu erzeugen,
wobei die Bilder erfasster Strömungsspitzen über die Zeit akkumuliert werden, um ein hochauflösendes Ultraschallbild zu erzeugen,
**dadurch gekennzeichnet, dass** die strömende Struktur aus strömendem biologischem Material eines untersuchten Subjektkörpers ohne jegliches hinzugefügtes Kontrastmittel oder jegliche hinzugefügte Mikrobläschen besteht.

2. Einrichtung nach Anspruch 1, wobei die strömende Struktur Erythrozyten beinhaltet, die erfassten Spitzen einer Strömung der Erythrozyten entsprechen und das hochauflösende Ultraschallbild die Gefäßstruktur visuell darstellt.

3. Einrichtung nach einem der Ansprüche 1 oder 2, wobei der Bewegungskorrektor der stationären Struktur die stationäre Struktur über die Reihe von Ultraschallbildern hinweg ausrichtet, um die Bewegung des Subjekts auszugleichen.

4. Einrichtung nach Anspruch 3, wobei der Bewegungskorrektor für stationäre Strukturen Bewegungsfelder einsetzt, um die stationäre Struktur zeitlich, räumlich oder zeitlich und räumlich auszurichten.

5. Einrichtung nach einem der Ansprüche 3 oder 4, wobei der Entferner stationärer Strukturen die stationäre Struktur von der bewegungskompensierten Reihe von Ultraschallbildern subtrahiert, um die Strömungsbilder zu erzeugen.

6. Einrichtung nach einem der Ansprüche 1 bis 5, wobei die Verarbeitungspipeline dazu konfiguriert ist, das hochauflösende Ultraschallbild in 1 bis 10 Sekunden zu erzeugen.

7. Einrichtung nach einem der Ansprüche 1 bis 6, ferner umfassend:
einen Strömungsverfolger (302), der dazu konfiguriert ist, die erfassten Strömungsspitzen über die Bilder erfasster Strömungsspitzen miteinander zu verknüpfen, wobei Strömungsspuren erstellt werden.

8. Einrichtung nach Anspruch 7, wobei der Strömungsverfolger dazu konfiguriert ist, Strömungsinformationen basierend auf den Strömungsspuren zu bestimmen.

9. Einrichtung nach einem der Ansprüche 7 bis 8, ferner umfassend:
ein Geschwindigkeitsschätzer (402), der dazu konfiguriert ist, Geschwindigkeitsinformationen basierend auf den Strömungsspuren zu schätzen.

10. Einrichtung nach einem der vorstehenden Ansprüche, die ein Wandlerarray mit einem oder mehreren Wandlerelementen zum Aussenden von Ultraschallwellen umfasst, die verwendet werden, um die Reihe von Ultraschallbildern zu erstellen, und
wobei das hochauflösende Ultraschallbild eine ausreichend hohe Auflösung aufweist, um Strukturen zu unterscheiden, deren Abmessungen kleiner als die halbe Wellenlänge der vom Wandlerarray ausgesendeten Ultraschallwellen sind.

11. Computerimplementiertes Verfahren, umfassend:
Bewegungskorrigieren stationärer Strukturen in einer Reihe von Ultraschallbildern hinsichtlich einer Subjektbewegung, wobei die Reihe von Ultraschallbildern die stationäre Struktur und eine strömende Struktur beinhaltet;
Entfernen der stationären Struktur aus der bewegungskorrigierten Reihe von Ultraschallbildern, wodurch Strömungsbilder der strömenden Struktur erzeugt werden;
Erfassen von Strömungsspitzen der strömenden Struktur in den Strömungsbildern über die Zeit, um Bilder erfasster Strömungsspitzen zu erzeugen; und
Akkumulieren der Bilder erfasster Strömungsspitzen über die Zeit, um ein hochauflösendes Ultraschallbild zu erzeugen,
**dadurch gekennzeichnet, dass** die strömende Struktur aus strömendem biologischem Material eines untersuchten Subjektkörpers ohne jegliches hinzugefügtes Kontrastmittel oder jegliche hinzugefügte Mikrobläschen besteht.

12. Verfahren nach Anspruch 11, wobei die strömende Struktur Erythrozyten beinhaltet, die erfassten Spitzen den Erythrozyten entsprechen und das hochauflösende Ultraschallbild die Mikrogefäßstruktur visuell darstellt.

13. Verfahren nach einem der Ansprüche 11 bis 12, wobei die Bewegungskorrektur Ausrichten der stationären Struktur über die Reihe von Ultraschallbildern hinweg beinhaltet, um die Bewegung des Subjekts auszugleichen.

14. Verfahren nach einem der Ansprüche 11 bis 13, wobei das hochauflösende Ultraschallbild eine ausreichend hohe Auflösung aufweist, um Strukturen zu unterscheiden, deren Abmessungen kleiner als die halbe Wellenlänge der Ultraschallwellen sind, die zum Erzeugen der Reihe von Ultraschallbildern verwendet werden.

15. Computerlesbares Speichermedium, das gespeicherte Anweisungen aufweist, die, wenn sie von einem Computer ausgeführt werden, den Computer veranlassen, die Schritte des Verfahrens nach einem der Ansprüche 11 bis 14 durchzuführen.

## Revendications

1. Appareil (106), comprenant :
un pipeline de traitement (124), incluant :
un correcteur de mouvement de structure stationnaire (204) configuré pour corriger le mouvement d'une structure stationnaire dans une série d'images ultrasonores pour le mouvement du sujet, dans lequel la série d'images ultrasonores inclut la structure stationnaire et la structure en mouvement ;
un dispositif de suppression de structures stationnaires (206) configuré pour supprimer la structure stationnaire de la série d'images ultrasonores corrigées du mouvement, produisant ainsi des images de flux de la structure en mouvement ; et
un détecteur de structure en mouvement (208) configuré pour détecter les pics de flux de la structure en mouvement dans les images de flux au fil du temps afin de générer des images des pics de flux détectés,
dans lequel les images des pics de flux détectés sont accumulées au fil du temps pour générer une image ultrasonore à super résolution,
**caractérisée en ce que** la structure de flux est constituée de matière biologique fluide d'un corps sujet en cours d'étude, sans un quelconque ajout d'agent de contraste ou de microbulles.

2. Appareil selon la revendication 1, dans lequel la structure de flux inclut des érythrocytes, les pics détectés correspondent au flux des érythrocytes et l'image ultrasonore à super résolution montre visuellement le système vasculaire.

3. Appareil selon l'une quelconque des revendications 1 à 2, dans lequel le correcteur de mouvement de la structure stationnaire aligne la structure stationnaire à travers la série d'images ultrasonores pour compenser le mouvement du sujet.

4. Appareil selon la revendication 3, dans lequel le correcteur de mouvement de la structure stationnaire utilise des champs de mouvement pour aligner la structure stationnaire temporellement, spatialement ou temporellement et spatialement.

5. Appareil selon l'une quelconque des revendications 3 à 4, dans lequel le dispositif de suppression de structure stationnaire soustrait la structure stationnaire de la série d'images ultrasonores compensées en mouvement pour produire les images de flux.

6. Appareil selon l'une quelconque des revendications 1 à 5, dans lequel le pipeline de traitement est configuré pour générer l'image ultrasonore à super résolution en 1 à 10 secondes.

7. Appareil selon l'une quelconque des revendications 1 à 6, comprenant en outre :
un dispositif de suivi de flux (302) configuré pour relier les pics de flux détectés à travers les images des pics de flux détectés, créant des pistes de flux.

8. Appareil selon la revendication 7, dans lequel le dispositif de suivi de flux est configuré pour déterminer les informations de flux en fonction des pistes du flux.

9. Appareil selon l'une quelconque des revendications 7 à 8, comprenant en outre :
un estimateur de vitesse (402) configuré pour estimer les informations de vitesse en fonction des pistes de flux.

10. Appareil selon l'une quelconque des revendications précédentes, comprenant un réseau de transducteurs comportant un ou plusieurs éléments transducteurs pour émettre des ondes ultrasonores utilisées pour créer la série d'images ultrasonores, et
dans lequel l'image ultrasonore à super résolution présente une résolution suffisamment élevée pour distinguer des structures dont les dimensions sont inférieures à la moitié de la longueur d'onde des ondes ultrasonores émises par le réseau de transducteurs.

11. Procédé mis en œuvre par ordinateur, comprenant :
la correction de mouvement d'une structure stationnaire dans une série d'images ultrasonores pour le mouvement du sujet, dans lequel la série d'images ultrasonores inclut la structure stationnaire et la structure en mouvement ;
la suppression de la structure stationnaire de la série d'images ultrasonores corrigées du mouvement, produisant ainsi des images de flux de la structure en mouvement ;
la détection des pics de flux de la structure en mouvement dans les images de flux au fil du temps afin de générer des images des pic détectés ; et
l'accumulation des images des pics de flux détectés au fil du temps pour générer une image ultrasonore à super résolution,
**caractérisée en ce que** la structure de flux est constituée de matière biologique fluide d'un corps sujet en cours d'étude, sans ajout d'agent de contraste ou de microbulles.

12. Procédé selon la revendication 11, dans lequel la structure de flux inclut des érythrocytes, les pics détectés correspondent aux érythrocytes et l'image ultrasonore à super résolution montre visuellement la microvasculature.

13. Procédé selon l'une quelconque des revendications 11 à 12, dans lequel la correction du mouvement inclut l'alignement de la structure stationnaire à travers la série d'images ultrasonores pour compenser le mouvement du sujet.

14. Procédé selon l'une quelconque des revendications 11 à 13, dans lequel l'image ultrasonore à super résolution présente une résolution suffisamment élevée pour distinguer des structures présentant des dimensions inférieures à la moitié de la longueur d'onde des ondes ultrasonores utilisées pour générer ladite série d'images ultrasonores.

15. Support de stockage lisible par ordinateur présentant des instructions qui, lorsqu'elles sont exécutées par un ordinateur, amènent l'ordinateur à effectuer les étapes de du procédé selon l'une quelconque des revendications 11 à 14.
